# EUROPEAN PATENT SPECIFICATION

(11) **EP 0 903 152 B1**
(45) Date of publication and mention of the grant of the patent: **12.11.2003**
(21) Application number: 98117466.7
(22) Date of filing: 15.09.1998
(51) Int. Cl.: A61K 47/48

(54) **Bifunctional water-soluble polymer derivative and complex containing it**
Bifunktionelle wasserlösliche Polymer-Derivate und diese enthaltende Komplexe
Dérivés polymeriques aquasolubles et complexes les contenant

(30) Priority: 17.09.1997 JP 25162497; 17.09.1997 JP 25162597
(43) Date of publication of application: 24.03.1999
(60) Divisional application: 03016154.1
(73) Proprietor: MITSUBISHI CHEMICAL CORPORATION, Chiyoda-ku, Tokyo 100-0005 (JP)
(72) Inventor: Tagawa, Toshiaki, Yokohama-shi, Kanagawa 227 (JP); Yada, Nobuhisa, Yokohama-shi, Kanagawa 227 (JP); Hirakawa, Yoko, Yokohama-shi, Kanagawa 227 (JP); Hosokawa, Saiko, Yokohama-shi, Kanagawa 227 (JP); Suzuki, Tsutomu, Machida-shi, Tokyo 194 (JP); Nagaike, Kazuhiro, Chiyoda-ku, Tokyo 100 (JP)
(74) Representative: TER MEER STEINMEISTER & PARTNER GbR

(56) References cited:
- EP-A- 0 520 499
- EP-A- 0 526 700
- EP-A- 0 607 978
- WO-A-94/22429
- WO-A-97/38731
- WO-A-98/41562
- WO-A-98/51336
- ALLEN T. ET AL: "A new strategy for attachment of antibodies to sterically stabilized liposomes resulting in efficient targeting to cancer cells" BIOCH. BIOPHYS. ACTA, vol. 1237, 1995, pages 99-108, XP002098995
- HASELGRUEBLER THOMAS HERMANN J_(A): "Synthesis and applications of a new poly(ethylene glycol) derivative for the crosslinking of amines with thiols." BIOCONJUGATE CHEMISTRY, 1995, pages 242-248, XP002098996
- DERKSEN J T P ET AL: "AN IMPROVED METHOD FOR THE COVALENT COUPLING OF PROTEINS TO LIPOSOMES" BIOCHIM BIOPHYS ACTA, vol. 814, 1985, pages 151-155, XP002098997
- SUZUKI S. ET AL: "Preparation of long-circulating immunoliposomes containing adriamycin by a novel method to coat immunoliposomes with poly(ethylene glycol)" BIOCHIMICA ET BIOPHYSICA ACTA,, vol. 1245, 1995, page 9-16 XP002098998
- MARUYAMA K. ET AL: "Targetability of PEG immunoliposomes conjugating antibodies at the terminal ends of PEG chains" POLYMER PREPRINTS,10 March 1997, pages 541-542, XP002098999
- PARR M. J. ET AL: "Accumulation of liposomal lipid and encapsulated doxorubicin in murine lewis lung carcinoma: the lack of beneficial effects by coating liposomes with poly(ethylene glycol)" J PHARMACOL EXP THERAP, vol. 280, no. 3, April 1997, pages 1319-1327, XP002099000

## Description

The present invention relates to a hetero bifunctional polymer derivative as a spacer to which a ligand (for example, a protein such as an antigen or an antibody, or a hormone) and an active substance selected from a liposome, may be bonded, and a complex thereby obtained, a pharmaceutical or diagnostic composition containing such a complex and a method for producing such a complex. The complex obtained by the present invention is useful as a carrier for a drug, or for diagnosis or examination.

In recent years, researches and developments have been made in various fields to impart specificity by bonding a ligand such as a hormone or a protein such as an antigen, an antibody or transferrin, to e.g. a drug, an isotope or a marker substance.

Particularly, it is expected to utilize a protein such as an antibody as a site-specific drug or a highly specific diagnostic agent by bonding it to a drug such as an anticancer drug, a radioisotope or a protein such as a toxin. Further, a complex having an antibody bonded to an enzyme such as alkaline phosphatase, has been practically used as a reagent for assay.

Such complexing is carried out by direct bonding or via a carrier such as a liposome. For example, in the case of an antibody, there may, for example, be a method wherein an antibody sugar chain is oxidized to an aldehyde and bonded to a hydrazide group-imparted active substance (such as an enzyme or a liposome), a method wherein a maleimido group is introduced to an amino group of the antibody, and a thiol group is introduced to an active substance, followed by bonding, a method wherein an endogenous disulfide group is reduced by a reducing agent to form a thiol, which is then reacted, and a method wherein it is bonded via avidin/biotin (Introduction to Enzyme Immunoassay, 3rd edition, Ishikawa et al., Igaku Shoin (1987)).

On the other hand, a polyethylene glycol (hereinafter sometimes referred to as "PEG") is a flexible water-soluble polymer and has been studied as a protein-modifier in many instances. PEG is known to have low toxicity and low immunogenicity, and it is known that by PEG-modification, protein retention in blood can be remarkably improved ("Progress of DDS 1995-1996", Nakayama Shoten, p82-94 (1995)).

Recently, a research has also been conducted wherein such PEG is used as a spacer as mentioned above. Particularly, in the liposome field, a research has been active on an antibody-bonded liposome using the PEG moiety as a spacer.

A lipid having a polar group, an amphipathic polymer or the like will form fine particles of e.g. a lipid micelle, a polymer micelle, a liposome or a lipid micelle closed double layer.

Many attempts have been made to utilize such fine particles as a carrier for a drug, nucleic acid or the like. With a liposome, a lipophilic substance can be included in the lipid phase, and a water-soluble substance can be included in the internal aqueous phase, and it is possible to support not only a low molecular weight compound but also a macromolecule such as a protein. Accordingly, many researches have been conducted to use it as a DDS (drug delivery system) carrier for a drug, a protein, nucleic acid, etc. In recent years, a research for practical application of a liposome having a targeting function or the like imparted by bonding e.g. an antibody or a sugar chain to the liposome surface, has been in progress, in addition to the effect of e.g. reducing toxicity by encapsulation of a drug.

On the other hand, it has been known that common drawbacks of a liposome, such as agglomeration and non-specific capture in the reticuloendothelial system, e.g. the liver or spleen, can be reduced by incorporating a water-soluble polymer such as polyethylene glycol, polyacrylamide, polyvinyl pyrrolidone or polyglycerol, to the liposome. Especially, with respect to the polyethylene glycol modification, many researches have been carried out, and it has been well known that the polyethylene glycol modification is effective to suppress the uptake in the reticuloendothelial system.

Further, a liposome is disclosed wherein in order to impart both a targeting function and a reticuloendothelial system-avoiding effect to a liposome, a targeting ligand such as an antibody is further bonded to a polyethylene glycol terminal of a polyethylene glycol-modified liposome.

Namely, a method of complexing an antibody and a liposome via a polyethylene glycol-lipid derivative having functional groups imparted, is disclosed, and the following references may be mentioned. Klibanov et al., J. Liposome Res. 2 (1992) 321, JP-A-6-126152, JP-A-6-220070, WO94/22429 Hansen et al., BBA 1239 (1995) 133 and Shahinian et al., BBA 1237 (1995) 99.

In these references, a lipid and a polyethylene glycol derivative are reacted in an organic solvent to synthesize a compound represented by lipid-PEG-functional group. Further, the compound is formed into a liposome together with other liposome forming lipids, followed by bonding to an antibody to obtain a complex of antibody-PEG-liposome.

As a specific polyethylene glycol-lipid derivative, JP-A-6-126152 discloses aliphatic hydrocarbon-PEG-carbonylimidazole; JP-A-6-220070 and BBA 1237 (1995) 99 disclose phosphatidylethanolamine (PE)-PEG-maleimide, and WO94/22429 and Hansen et al., BBA 1239 (1995) 133, disclose an antibody-bonded liposome employing PE-PEG-NH₂-Biotin, distearoylphosphatidylethanolamine (DSPE)-PEG-hydrazide (Hz) or DSPE-PEG-3-(2-pyridylthio)propionyl (PDP).

It has been demonstrated that by such complexing, the coupling efficiency of the antibody can be improved, and the kinetics in blood can be improved.

However, at the same time, some problems have been pointed out. Namely, it has been found that by the method wherein DSPE-PEG-Hz is used, the activity of the antibody decreases during the treatment of the antibody and the coupling efficiency of the antibody is not very much improved. In the case of DSPE-PEG-PDP, reduction treatment is necessary after liposome-formation, which may affect the encapsulated drug, and the operation is cumbersome. With PE-PEG-NH₂-Biotin, bonding is via avidin/biotin, whereby in its administration in vivo, immunogenicity is likely to appear, as the avidin/biotin may be taken as a foreign protein. Further, with aliphatic hydrocarbon-PEG-carbonylimidazole, a problem has been pointed out that the PEG derivative is likely to react with an amino group of the drug to be included.

Further, in order to bond the antibody with adequate efficiency, an excess amount, relative to the antibody, of the PEG-lipid derivative has to be incorporated into the liposome. Accordingly, even after bonding of the antibody, PEG moieties having active groups will remain excessively in the boundary region where they are readily reactive with other components (biological components in the case of in vivo administration). Further, it has been reported that if such an excess amount of a lipid-PEG-functional group derivative is incorporated into a liposome, leakage of the substance included in the liposome tends to be accelerated (BBA 1237 (1995) 99).

From such viewpoints, the prior art has not necessarily been fully satisfactory.

Allen et al. (1995) BBA 1237, 99-108, and Haselgruebler et al. (1995) Bioconjug. Chem., 242-248, disclose bifunctional polymers having a pyridyldithiopropionyl (PDP) moiety at one end which contain a structure represented by PDP-PEG. PDP has a terminal structure represented by pyridyl-S-S- and does not contain an acetylthio group.

Maruyama et al. (1997) Polymer Preprints, 541-542, discloses a bifunctional polymer represented by DPPE-PEG-maleimido which has a dipalmitoyl phosphatidyl ethanolamine residue at one end. The bifunctional polymer does not comprise an acetylthio group.

WO 9738731 A and WO 9422429 A disclose bifunctional polymers, DSPE-PEG-maleimido, having a distearoylphosphatidylethanolamine, but not an acetylthio group, at one end.

EP-A-0607978 discloses a bifunctional polymer (PEG) having maleimide moieties at both ends, but not an acetylthio group.

Blume et al. (1993) BBA 1149, 180-184, discloses bifunctional polymers, DSPE-PEG-COOH not containing a thiol group, to attach targeting substances to immunoliposomes.

The present inventors have conduced an extensive study to solve such conventional problems and as a result, have found it possible to overcome the conventional problems by using, as a spacer, a hetero bifunctional water-soluble polymer which is a synthetic water-soluble polymer derivative and which has a succinimide group as a moiety reactive with an amino group and a latent thiol group as a S-acetylthio group.

Further, they have found a method for readily producing a ligand-bonded complex, wherein the hetero bifunctional water-soluble polymer derivative is firstly bonded to a ligand and then bonded, at the other end, to an active substance selected from a liposome, and further, if necessary, a monovalent water-soluble polymer having reactivity with the active substance, is bonded to the active substance.

Namely, the present invention provides a bifunctional water-soluble polymer derivative as defined in claim 1 having a succinimide group as a moiety reactive with an amino group and a S-acetylthio group as a latent thiol group moiety. The water-soluble polymer derivative is preferably represented by the following formula (I): 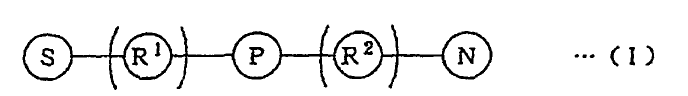 wherein S is said S-acetylthio group moiety, P is a water-soluble polymer, N is said succinimide group reactive with an amino group, and each of R¹ and R² is an optional linking group, but R¹ and/or R² is not essential. In the polymer derivative, the water-soluble polymer is preferably a biodegradable polymer or a polyethylene glycol, more preferably a polyethylene glycol.

The present invention also provides a complex having a ligand and an active substance selected from a liposome bonded via such a water-soluble polymer derivative. The complex is preferably represented by the following formula (II): 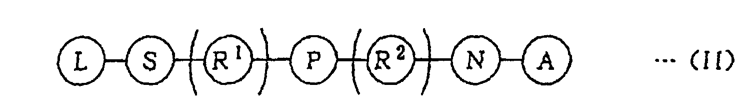 wherein L is said liposome, A is a ligand, and S, R¹, P, R² and N are as defined above. The ligand is preferably an antibody, more preferably an antibody 1-3-1. A monofunctional water-soluble polymer derivative having reactivity with the active substance may be bonded to the active substance.

Further, the present invention provides a pharmaceutical composition such as an antitumor agent, or a diagnostic composition, which contains such a complex.

Still further, the present invention provides a method for producing such a complex, which comprises steps of bonding the above mentioned bifunctional water-soluble polymer derivative to a ligand and then bonding it, at the other end of the water-soluble polymer derivative, to an active substance selected from a liposome. The method may further contain a step of bonding a monofunctional water-soluble polymer derivative having reactivity with the active substance, to the active substance. In the method, the bifunctional water-soluble polymer derivative and the ligand are bonded preferably via an amino group of the ligand. Further, in the method, the bifunctional water-soluble polymer derivative and the active substance are bonded such that a moiety reactive with a thiol group of the active substance is bonded to a thiol group moiety of the bifunctional reactive water-soluble polymer derivative.

Namely, the water-soluble polymer of the present invention (hereinafter sometimes referred to as "the present polymer compound" or "the present water-soluble polymer") is bonded firstly to a ligand to form a thiol group-imparted PEG-ligand complex (in the case of a S-acetylthio group, after deacetylation under a mild condition) and then mixed and reacted with an active substance selected from a liposome having a moiety reactive with thiol imparted, whereby a ligand-PEG-active substance complex can readily be obtained.

According to this method, oxidation or reduction which is likely to bring about deterioration of the activity of a ligand such as an antibody, is not required. Further, it has been made possible to form the complex by a reaction via a thiol group which is scarcely influential over the active substance.

It has been surprisingly found that when, for example, an antibody and a liposome are bonded via the present polymer compound, a high bonding activity to a target cell is shown as compared with a case where bonding is carried out via a low molecular weight compound having the same combination of functional groups, which does not have a polymer moiety. Further, an effect for reducing leakage of the included substance which has been heretofore pointed out, has been found.

Further, according to the method of the present invention, a ligand (such as an antibody)-PEG derivative is firstly formed, whereby it is possible to form a ligand-PEG complex without introducing an excess amount of a functional PEG-lipid derivative to a thermodynamically instable active substance (such as liposomes) as in the conventional method. Accordingly, it is possible to form an active substance by adequately utilizing the conventional techniques for the production of an active substance such as a liposome or for encapsulation of a drug or the like, without being influenced by the adverse effect which is likely to result due to the conventional functional group-PEG-lipid.

Further, the method of the present invention is free from the problem of the conventional method such that after bonding of the ligand, PEG moieties having active groups tend to remain excessively in the vicinity of the boundary where they are readily reactive with other components (biological components in the case of in vivo administration).

Further, by using the hetero bifunctional water-soluble polymer derivative, it is unnecessary to carry out oxidation or reduction which is likely to bring about deterioration of the activity of the ligand such as an antibody.

Now, the present invention will be described in detail.

The water-soluble polymer derivative of the present invention is bifunctional. In the present invention, "bifunctional" means that there are two groups reactive with functional groups, in the same molecule, and they are different from each other.

The water-soluble polymer used in the present invention, is a synthetic polymer such as a polyethylene glycol, a polyacrylamide, a polyvinylpyrrolidone, a polyglycerol, a polylactic acid, a polyglycolic acid or a polyamino acid, preferably a polyethylene glycol. Further, a biodegradable polymer such as a polyamino acid and polyhydroxy acid, may suitably be used. The molecular weight is preferably from about 500 to 20,000, more preferably from 1,500 to 10,000, most preferably from 2,000 to 6,000.

The water-soluble polymer derivative of the present invention has a S-acetylthio group moiety as a latent thiol moiety at one terminal end of the above-mentioned water-soluble polymer and a succinimide group as a moiety reactive with an amino group at the other end.

The latent thiol moiety is a protected thiol moiety, i.e. an acetylthio group moiety, and can be deblocked under suitable procedure if necessary.

The latent thiol group may be bonded directly to the water-soluble polymer or may be bonded via a linking group such as an alkylene, carbonyl, ester or amido group interposed therebetween.

The succinimide group as a moiety reactive with an amino group means a moiety, of which a carboxyl group or a hydroxyl group is capable of condensation reaction with the amino group. Specifically, such can be accomplished by means of e.g. a succinimide ester. The moiety reactive with an amino group may also be bonded directly to the water-soluble polymer or may be bonded via a linking group as mentioned above.

The water-soluble polymer derivative of the present invention is not particularly limited, but may, for example, be obtained by reacting mercaptoethylamine with a polyethylene glycol activated with an N-hydroxysuccinimide ester. Also it may be obtained by reacting a polyethylene glycol having an amino group and a carboxy group with an N-hydroxysuccinimide ester of S-acetylthioglicolic acid (SATA), an N-hydroxysuccinimide ester of S-acetylthiopropionic acid or S-acetylthioacetic acid anhydride to introduce S-acetylthio group moiety, followed by reacting with N-hydroxysuccinimide and N,N'-dicyclohexylcarbodiimide or p-nitrophenyl trifluoroacetate to activate the carboxyl group.

The bifunctional water-soluble polymer derivative of the present invention obtained as described above, can form a complex having a ligand and an active substance bonded thereto.

The ligand preferably has an amino group and will bond to the moiety reactive with an amino group, of the water-soluble polymer of the present invention. The ligand may, for example, be a lectin, an antigen, an antibody, a hormone or a transmission substance, preferably an antibody, more preferably an antibody 1-3-1. Further, it is also possible to use a ligand as described hereinafter with reference to a method for producing the complex.

Bonding of the ligand having an amino group to the water-soluble polymer can easily be accomplished by mixing the ligand and the water-soluble polymer in an aqueous solution and reacting them at room temperature. The molar ratio of the polymer derivative to the ligand is preferably from about 0.3 to 50, more preferably from 0.8 to 30.

When the polymer having a S-acetylthio group imparted, is used as the water-soluble polymer, it may be bonded to the ligand as described above, followed by deacetylation to form a thiol group, and then it is subjected to bonding with an active substance as described below. The condition for deacetylation is not particularly limited so long as it is a mild condition under which the ligand is substantially stable. Preferably, the deacetylation can be accomplished by a reaction employing hydroxylamine in a neutral buffer solution at room temperature so that the final concentration will be within a range of from 5 to 200 mM, preferably from 20 to 200 mM, more preferably from 20 to 100 mM.

After bonding a ligand as described above, an active substance selected from a liposome can be bonded to the other end of the water-soluble polymer.

Further, a drug or a diagnostic agent may be included in the liposome.

The drug which may be included in the liposome includes, for example, an antitumor agent such as adriamycin, daunomycin, vinblastin, cisplatin, mitomycin, bleomycin, actinomycin or 5-FU (fluorouracil), an adrenergic blocker such as timolol, a hypertension drug such as clonidine, an antiemetic agent such as procainamide, an antimalarial agent such as chloroquine, an antibiotic such as amphotericin, as well as pharmaceutically acceptable salts and derivatives thereof; a toxic protein such as ricin A or diphtheria toxin, or DNA coding it, DNA coding a gene of a cytokine such as TNF and a nucleotide such as antisense DNA. As the pharmaceutically acceptable salt of the above antitumor agent or the like, a salt with a polyvalent anionic substance, such as *a* citric acid salt, a tartaric acid salt or a glutamic acid salt, is preferred. Particularly preferred is an antitumor agent.

The diagnostic agent may, for example, be an imaging agent such as a radioactive element such as indium or technetium; MRI contrast agent such as gadolinium compounds; X ray contrast agent such as iodine compounds; ultrasonic contrast agent such as carbon dioxide; an enzyme such as horseradish peroxidase or alkaline phosphatase; a fluorescent substance such as an europium derivative or carboxyfluorescein; a light-emitting substance such as an N-methylacridium derivative.

Introduction of such a drug or a diagnostic agent into a liposome can be carried out by a method per se commonly known. For example, a method may be employed wherein a concentration gradient such as a pH gradient is formed at the time of forming a liposome, and an ionizable drug is taken up using this potential as a driving force (Cancer Res. 49 5922 (1989)).

To the active substance, a moiety reactive with a thiol group is imparted by a known method. Namely, a reactive group selected from e.g. a maleimido group, an alkyl halide group, an aziridine group and a pyridyldithio group, may be used. However, preferred is a maleimido group. As a specific example which by no means restricts the present invention, a liposome having a maleimido group moiety can be obtained by forming into a liposome, a lipid having a maleimido group moiety such as maleimidocaproyldipalmityl phosphatidylethanolamine (JP-A-4-346918) or maleimidophenylbutyroyl phosphatidylethanolamine together with phosphatidylcholine or cholesterol in accordance with a known method (Liposome, Chapter 2, editted by Nojima et al., Konando (1988)). As the liposome, various liposomes such as MLV, LUV and SUV, can be employed. However, preferred is LUV.

Further, a protein having a maleimido moiety can be obtained by reacting N-succinimidyl-6-maleimidohexanoate with a protein. Further, a fluorescent dye such as maleimido fluorescein or eosine maleimide may also be used.

The active substance having such a thiol-reactive group imparted, can readily be complexed by mixing it with the water-soluble polymer-ligand complex having a thiol group obtained as described above, in a buffer solution close to neutral, whereby a complex composed of the desired ligand-PEG-active substance, can be obtained. Further, the product may be purified by such a means as ultrafiltration, gel permeation chromatography, ion exchange chromatography, as the case requires.

In the accompanying drawings:

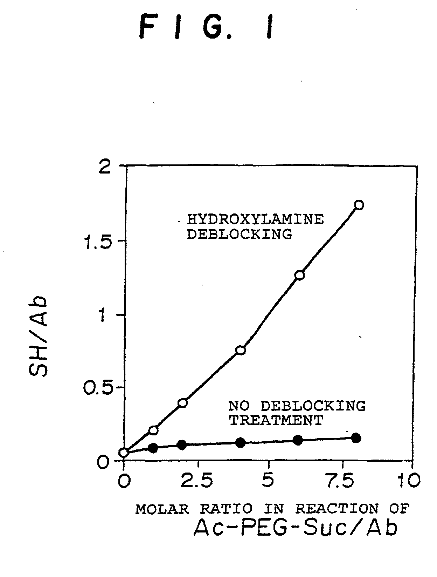

Figure 1 illustrates introduction of Ac-S-PEG-Suc to an antibody and formation of a thiol group by deblocking. The abscissa indicates the molar ratio of Ac-S-PEG-Suc to the antibody at the time of the reaction, and the ordinate indicates the formed thiol group molecular number per molecule of the antibody. White dots represent the antibody deblocked with hydroxylamine after the reaction with Ac-S-PEG-Suc, and black dots represent the antibody which was not deblocked with hydroxylamine.

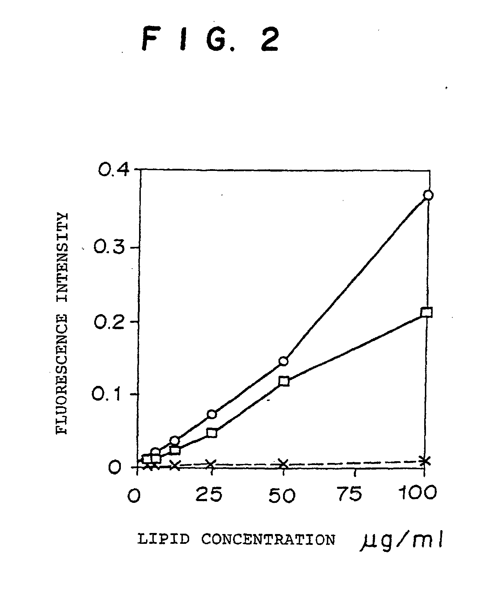

Figure 2 illustrates the bonding activity of a carboxyfluorescein-encapsulated liposome having bonded an anti-CEA antibody having a thiol group introduced by means of Ac-S-PEG-Suc or SATA. The respective immunoliposomes and a liposome having no antibody bonded were reacted with CEA-fixed plates, and after washing, the amounts of liposomes bonded to the plates were measured by fluorescence. The abscissa indicates the amounts of the respective liposomes (lipid amounts), and the ordinate indicates the fluorescence intensity at an emission wavelength of 520 nm with an exciting wavelength of 492 nm. Circular dots represent an immunoliposome using an antibody having a thiol group introduced by means of Ac-S-PEG-Suc, square dots represent an immunoliposome using an antibody having a thiol group introduced by means of SATA, and × dots represent a liposome having no antibody bonded.

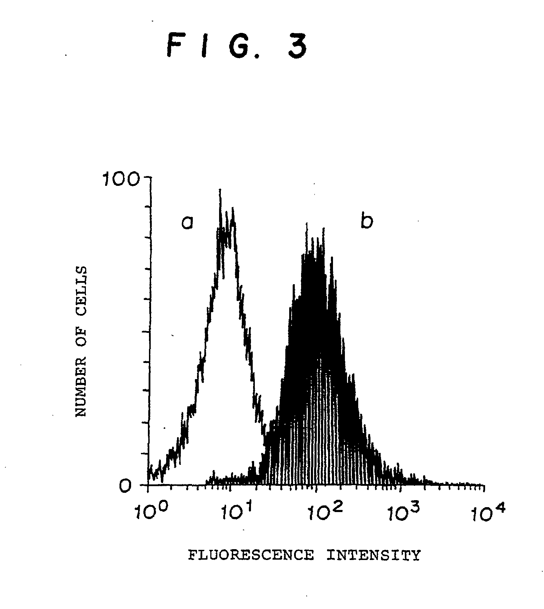

Figure 3 illustrates the bonding activities to MKN 45 cancer cells, of a fluorescent antibody prepared by fluorescein maleimide and an antibody having a thiol group introduced by means of Ac-S-PEG-Suc. After reaction with cells, the analysis was carried out by a flow cytometer. In the Figure, a represents a group of cells not reacted with the fluorescent antibody, and b represents a group of cells reacted with the fluorescent antibody. The abscissa indicates the fluorescence intensity, and the ordinate indicates the number of cells.

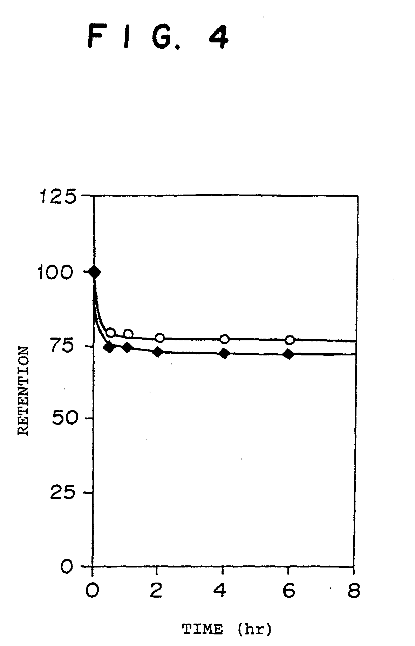

Figure 4 shows a comparison between leakage of included carboxyfluorescein from an immunoliposome (white dots) employing Ac-S-PEG-Suc and leakage from a non-modified liposome (black square dots). The ordinate represents % of carboxyfluorescein remaining in the liposome, and the abscissa indicates the incubation time.

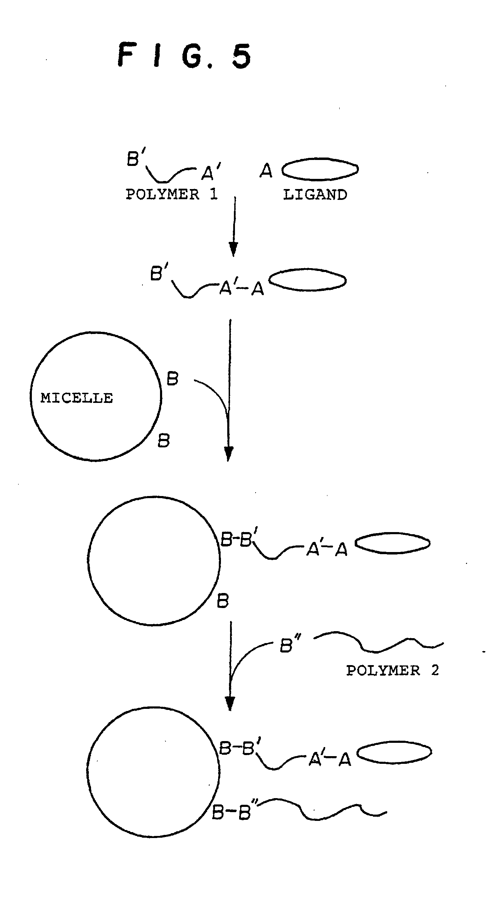

Figure 5 illustrates a method for producing a ligand-bonded micelle according to the present invention. A is a functional group in the ligand, A' is a group having reactivity with A, B is a reactive group introduced to a micelle particle and has specific reactivity with B' and B".

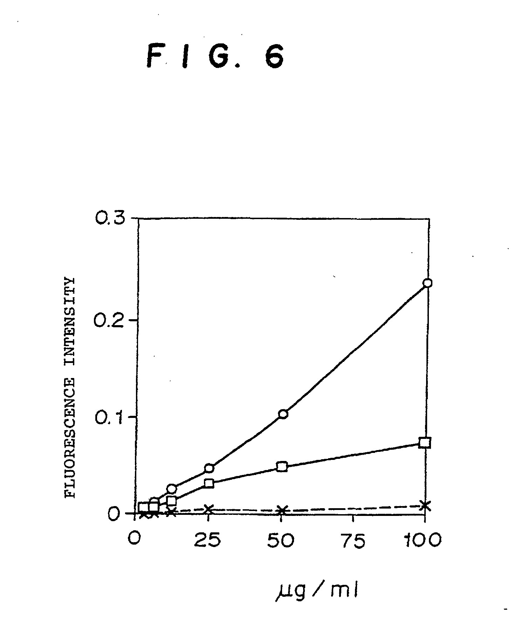

Figure 6 shows the bonding activities of carboxyfluorescein-encapsulated liposomes having bonded an anti-CEA antibody having a thiol group introduced by means of Ac-S-PEG-Suc or SATA and further coated with a thiol-modified polyethylene glycol. The abscissa indicates the amounts of the respective liposomes (lipid amounts), and the ordinate indicates a fluorescence intensity at an emission wavelength of 520 nm with an exciting wavelength of 492 nm. Circular dots represent an immunoliposome employing an antibody having a thiol group introduced by means of Ac-S-PEG-Suc, square dots represent an immunoliposome employing an antibody having a thiol group introduced by means of SATA, and × dots represent a liposome having no antibody bonded.

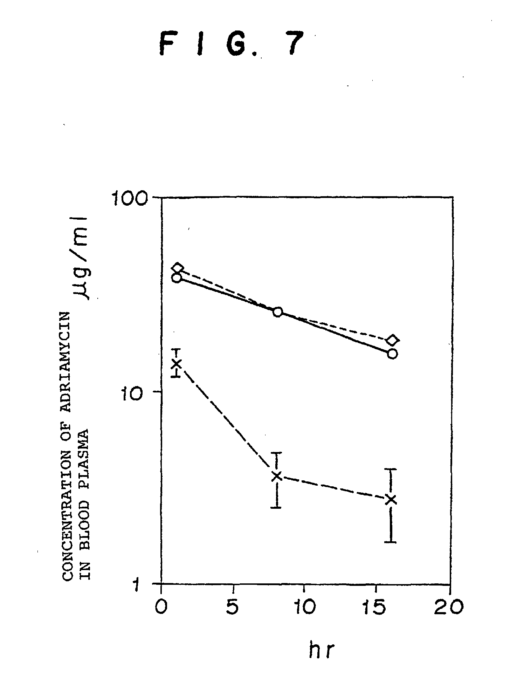

Figure 7 shows a comparison of changes in the concentration in blood. The ordinate indicates the concentration of adriamycin in blood plasma, and the abscissa indicates the time. Each dot represents an average of a group of four and SD (standard deviation). In the Figure, circular dots represent an immunoliposome employing an antibody having a thiol group introduced by means of Ac-S-PEG-Suc, square dots represent a liposome having only thiol-modified PEG bonded, and × dots represent a simple liposome having no thiol-modified PEG bonded.

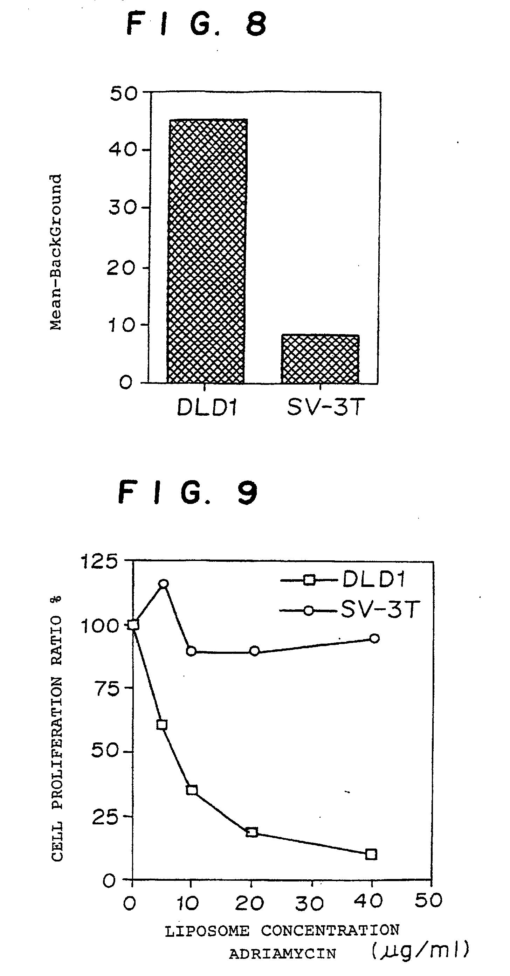

Figure 8 is a graph showing the reactivities of 1-3-1 antibody against DLD-1 cells and SV-3T cells. The ordinate represent a value obtained by subtracting a value in a case where no antibody is contained, as a background value, from an average channel number.
Figure 9 is a graph showing the cell proliferation inhibitory activities of the immunoliposome obtained in Example 8 against DLD-1 cells and SV-3T cells. The ordinate indicates the number of cells at the time of addition of the liposome sample (absorbance in MTT assay) relative to the control being 100%. The abscissa indicates the liposome as the concentration of adriamycin. Symbol □ indicates DLD1 cells, and symbol ○ represents the proliferation ratio of SV-T3.

Now, the method for producing the complex will be described in detail.

Figure 5 illustrates the outline of the method of the present invention. In the Figure, polymer 1 is the above mentioned bifunctional water-soluble polymer derivative comprising an A' moiety having reactivity with a functional group A in a ligand and a B' moiety to be bonded to a reactive moiety B incorporated in an active substance. Polymer 2 has B" to be bonded to B. B' and B" may be the same residues. Thus, in the present invention, a monofunctional polymer having reactivity with an active substance, may be bonded.

In the present invention, firstly a hetero bifunctional water-soluble polymer 1 is bonded to a ligand, and then bonded via its other end, to an active substance. Further, a monofunctional polymer 2 having reactivity with the active substance may further be bonded to obtain a complex.

As A in the ligand, an amino group or a sugar chain moiety may be employed, but preferred is an amino group. A' as a group reactive with an amino group is as described above.

The combination of B and B' is selected from combinations whereby the B-B' bond is specifically formed with no substantial reaction of B with the ligand. However, a combination is preferred wherein B' is a thiol group, and B is a group reactive with a thiol group. The thiol group may be a protected latent thiol group i.e. a S-acetylthio group, before the reaction, as mentioned above. In such a case, the S-acetylthio group is subjected to deblocking at the time of the reaction.

The ligand to be used in the present invention may be a ligand selected from a protein such as transferrin, EGF or α-fetoprotein (AFP), a peptide such as insulin and an antibody such as a polyclonal antibody or a monoclonal antibody, in addition to those mentioned above, and it may be in its entirety or may be its fragment obtainable by e.g. enzymatic treatment. When it is used for treatment of various diseases, it is preferably a mouse-human chimera antibody or a human antibody.

The water-soluble polymers 1 and 2 may be those mentioned above. The polymers 1 and 2 may be in a different combination or the same combination.

The reaction of the ligand and the polymer 1 is as described above.

As mentioned above, in a case where a polymer having a S-acetylthio group imparted, is used, after it is bonded to the ligand, the S-acetylthio group may be deacetylated to a thiol group.

In the present invention, the active substance may be as mentioned above. In the following description, the complex will be described sometimes with reference to a micelle or a liposome as a typical example of the active substance.

The micelle is composed of amphipathic molecules. As amphipathic molecules constituting the micelle, any molecules may be employed so long as they contain hydrophilic moieties and hydrophobic moieties, and they are capable of forming a micelle by a method which is per se commonly known. Lipids may be mentioned as preferred amphipathic molecules among them.

The lipids capable of forming micelles in the present invention may, for example, be natural phosphatidylcholine such as egg yolk phosphatidylcholine (EYPC); a phosphatidylcholine (PC), such as dipalmitoyl phosphatidylcholine (DPPC), dimyristoyl phosphatidylcholine (DMPC), distearoyl phosphatidylcholine (DSPC) or dioleoyl phosphatidylcholine (DOPC); natural phosphatidylethanolamine such as egg yolk phosphatidylethanolamine (EYPC); a phosphatidylethanolamine (PE) such as dipalmitoyl phosphatidylethanolamine (DPPE) or dioleoyl phosphatidylethanolamine (DOPE); a phosphatidylglycerol (PG), such as dipalmitoyl phosphatidylglycerol (DPPG); phosphatidylserine (PS); a phosphatidylinositol (PI); and a phospholipid such as phosphatidic acid (PA), a sphingoglycolipid, or a glyceroglycolipid. These lipids may be used alone or in combination as a mixture of two or more of them, or in a combination of such lipids with a nonpolar substance such as cholesterol.

To the micelle in the present invention, in addition to the above lipid and the nonpolar substance, a lipid derivative to be subjected to bonding to the ligand-polymer 1 composite or to the polymer 2, is incorporated. As such a lipid derivative, it is possible to employ a phospholipid derivative having a reactive group selected from a maleimido group, an alkyl halide group, an aziridine group and a pyridyldithio group, as a reactive moiety B incorporated in the micelle i.e. as the moiety reactive with a thiol group, but the reactive group is preferably a maleimido group. As a specific example which by no means restricts the present invention, it is possible to employ a lipid having a maleimido group moiety such as maleimidocaproyldipalmityl phosphatidylethanolamine (JP-A-4-346918) or maleimidophenylbutyroyl phosphatidylethanolamine.

The above lipid derivative can be used usually in an amount of from 0.1 to 8 mol%, preferably from 0.5 to 3 mol%, more preferably from 1 to 3 mol%, to the micelle-forming amphipathic molecules. Cholesterol can be used in an amount of from 0 to 100 mol%, preferably from 40 to 70 mol%, to the micelle-forming amphipathic molecules.

The micelle in the present invention may be one produced by any method and can be produced by using the above mentioned materials by means of a technique which is per se commonly known. For example, it is possible to employ a multilamellar liposome (MLV) formed by adding an aqueous solution to a lipid thin film deposited on a glass wall and exerting mechanical-shaking, a small unilamellar liposome (SUV) obtained by an ultrasonic treating method, an ethanol injection method or a French press method, or a large unilamellar liposome (LUV) obtained by e.g. a surfactant removing method, a reversed phase evaporation method (Liposome, Junichi Sunamoto et al., Nankodo, 1988) or an extrusion method wherein MLV is excluded through a membrane having a uniform pore size (Liposome Technology, Vol. 1, 2nd Edition).

Further, to the micelle, a drug or a diagnostic agent may be included. The drug or the diagnostic agent to be included in the micelle is as described above.

The reaction of the ligand-polymer 1 complex with the above liposome or micelle can easily be accomplished by mixing them in a neutral buffer solution at a temperature of from about 4 to 40°C. Further, in a case where the thiol is protected, it may be deblocked prior to the reaction, as mentioned above.

The pH during the reaction is preferably from about 5 to 8, more preferably from 5.5 to 7.0. During the reaction, about 1 mM of EDTA may be added.

The ligand-polymer 1 complex may be used for the reaction in an amount of at most equivalent to the reactive lipid incorporated in the micelle. In a case where the desired targeting ability can be obtained with a small amount of a ligand, it is not necessary to bond a large amount of the ligand, and polymer 2 may be added and reacted to the remaining reactive groups on the micelle. Further, by the addition of polymer 2, retention in blood of the micelle-polymer 1-ligand complex can be secured when it is used, for example, as a drug or a diagnostic agent.

In the present invention, polymer 2 is as described above. Polymer 2 has substantially only a site for covalent bonding to the micelle.

Although not particularly limited, as polymer 2, for example, a PEG derivative having a thiol group comprising cysteine and 2,4-bis(polyethylene glycol)-6-chloro-S-triazine (JP-A-4-346918) or N-propionate-3-(thiol)methoxypolyoxyethyleneamine (J. Controlled Release 28 (1994) 155) may be employed.

Polymer 2 may be reacted after the reaction of the micelle with the ligand-polymer 1 complex, or it may be added after once purifying the micelle-ligand-polymer 1 complex.

It may be added in an amount of from 0.2 to 5 mol, preferably from 0.5 to 2 mol, per mol of the reactive lipid incorporated in the micelle, and the reaction can readily be accomplished by simply mixing them in a neutral buffer solution at a temperature of from 4 to 40°C.

The pH during the reaction is preferably from 5 to 8, more preferably from 5.5 to 7.0.

During the reaction, about 1 mM of EDTA may be added.

The ligand-bonded micelle obtained by the present invention may further be purified by a method such as ultrafiltration, gel permeation chromatography, before use, as the case requires.

Now, the present invention will be described in further detail with reference to Examples. However, it should be understood that the present invention is by no means restricted to such specific Examples.

### EXAMPLE 1

To 1 g of poly(ethylene glycol)-bis-ω-amino-α-carboxyl (PEG average molecular weight: 3400, Shearwater polymers, Inc) dissolved in 10 ml of methylene chloride, 74.8 mg of S-acetylthioglycolic acid N-hydroxysuccinimide ester (Sigma) and 41 µl of triethylamine were added, stirred and dissolved. Then, 10 mg of S-acetylthioglycolic acid N-hydroxysuccinimide ester was further added, followed by stirring for reaction at room temperature for 3.5 hours. The progress of the reaction was confirmed by TLC (chloroform/methanol = 85/10, iodine color development, hereinafter TLC was carried out under the same conditions) by a shift of the spot of low Rf of poly(ethylene glycol)-bis-ω-amino-α-carboxyl added to high Rf (about 0.6).

The solvent was distilled off in a nitrogen atmosphere, and the reaction product was dissolved by an addition of 10 ml of chloroform. The solution was added to sep-pak (SILICA PLUS Wates) swelled with chloroform and eluted with chloroform/methanol (4/1 (v/v)) to pretreat the sample. The solvent was again distilled off in a nitrogen atmosphere, and the product was dissolved in chloroform. Then, the solution was added to a silica gel column (Rover column, LiChroprep Si60 25×310 cm, manufactured by Kanto Kagaku). After washing with chloroform, the product was developed and eluted with chloroform/methanol (85/15 (v/v)) to obtain a main product with Rf of TLC being about 0.6, which was purified. The solvent was distilled off in a nitrogen atmosphere, and 583 mg of the product was obtained. 543 mg thereof was dissolved in about 2 ml of dehydrated methylene chloride, precipitated by an addition of diethyl ether, collected by filtration and dried under reduced pressure by using a vacuum pump. The product was dissolved in 5 ml of dehydrated methylene chloride, and 17.8 mg of N-hydroxysuccinimide (Sigma) was added, followed by stirring for 10 minutes. Further, 31.9 mg of N,N'-dicyclohexylcarbodiimide was added thereto, followed by reaction overnight at room temperature in a nitrogen atmosphere with stirring. The precipitate was separated by filtration, and the solvent was distilled off in a nitrogen atmosphere. The product was dissolved in a small amount of dehydrated methylene chloride. Ethyl ether was added thereto, whereupon the formed precipitate was collected by filtration and dried under reduced pressure by using a vacuum pump and subjected to TLC to obtain 337 mg of the uniform desired product (hereinafter referred to as "Ac-S-PEG-Suc"). The desired product was confirmed by ¹H-NMR.

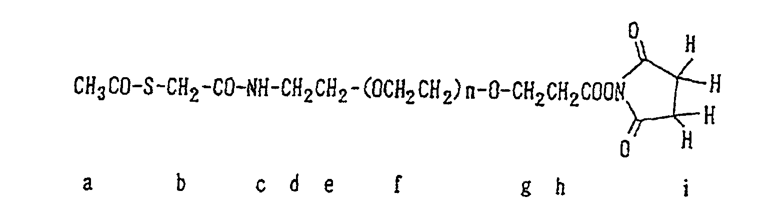

**Table 1**

| ppm | | | Attribution |
|---|---|---|---|
| 2.38 | s | 3H | a |
| 2.80 to 2.85 | m | 4H | i |
| 2.88 | t | 2H | h |
| 3.40 | dd | 2H | d |
| 3.52 | dd | 2H | e |
| 3.55 | s | 2H | b |
| 3.60 | - | - | f |
| 3.82 | t | 2H | g |
| 6.65 | s | 1H | c |

Further, formation of the desired product was confirmed by the bonding experiment to human γ-globulin.

Namely, 30 mg/ml of Ac-S-PEG-Suc dissolved in dehydrated methanol was added to 0.9 ml of human γ-globulin (IgG, F(ab')₂) (4.7 mg/ml) dissolved in a buffer solution comprising a 50 mM phosphorate buffer solution (pH 7.0) and 1 mM EDTA. The added amount was from 0 to 8 times by molar ratio to the antibody, and the relation between the added amount and the amount of PEG introduced was examined. After reacting at 25°C for one hour upon the addition of Ac-S-PEG-Suc, the product was purified by gel chromatography by developing with the above mentioned buffer solution using Sephacryl S100 (Pharmacia) to separate unreacted Ac-S-PEG-Suc (PEG molecular weight: 3.4 K) and the antibody (molecular weight: 100 K).

A part of the obtained modified antibody was deblocked with hydroxylamine. Namely, 0.1 ml of a hydroxylamine solution (0.5 M hydroxylamine, 0.5 M HEPES, 25 mM EDTA, pH 7.0) was added to 0.9 ml of the antibody solution, and reacted at 25°C for 10 minutes, followed by deacetylation, and then low molecular weight molecules were removed by PD-10 (Pharmacia) equilibrated with a 0.1 M phosphate buffer solution of pH 6.0 and 1 mM EDTA to obtain an antibody having a thiol group. The thiol group introduced to the antibody was measured by a method employing 4,4'-dithiopyridine (Sigma) (Continued Biochemistry Experiment Lecture 5, p 109, compiled by Japan Biochemistry). As a control, the thiol content of each modified antibody which was not subjected to deblocking treatment with hydroxylamine, was measured in the same manner. As a result, as shown in Figure I, the human γ-globulin modified by Ac-S-PEG-Suc was found to have a latent thiol group formed by deblocking with hydroxylamine i.e. a S-acetylthio group, and it was confirmed that such a latent thiol group increases depending upon the amount of addition of Ac-S-PEG-Suc.

### EXAMPLE 2

In the same manner as in Example 1, Ac-S-PEG-Suc was reacted to anti-CEA mouse monoclonal antibody (IgG₁, F(ab')₂) to obtain a modified antibody, and then the antibody protein was purified by a cation exchange resin. Namely, the reaction solution (3.4 mg/ml 2 ml) was adjusted to pH 4 by an addition of 0.1 M acetic acid and added to 2 ml bed of SP sepharose (Pharmacia) equillibrated with a 0.1 M acetate buffer solution of pH 4.0. After washing with 4 ml of the same buffer solution, the protein was eluted with a 50 mM phosphate buffer solution (pH 7.5). By means of Centricon 30 (Amicon) ultrafilter, the buffer solution was adjusted to a 50 mM phosphate buffer solution (pH 7.0) containing 1 mM EDTA, and deblocking was carried out by an addition of hydroxylamine in the same manner as in Example 1. The SH group-imparted antibody was subjected to desalting by PD-10 and exchanged with a 0.1 M phosphate buffer solution of pH 6.0 and 1 mM EDTA solution, and then used for bonding to a liposome as described hereinafter. Further, Ac-S-PEG-Suc and Ac-S-PEG-COOH (compound prior to succinimido-modification in Example 1) which corresponds to a hydrolyzate of Ac-S-PEG-Suc did not bond to SP sepharose under the same condition.

Further, as a control, instead-of Ac-S-PEG-Suc, S-acetylthioglycolic acid N-hydroxysuccinimide ester (Sigma) having no PEG moiety between the S-acetyl group and the succinimide group (hereinafter sometimes referred to as "SATA") was reacted with the same antibody, followed by desalting by PD-10, hydroxylamine deblocking, and PD-10 desalting (0.1 M phosphate buffer of pH 6.0 and 1 mM EDTA) to obtain an antibody having a thiol group imparted.

Using a liposome having a fluorescent dye carboxyfluorescein encapsulated and having, as a membrane component, a maleimido-modified lipid having reactivity with thiol, bonding with the above antibody was studied.

Namely, 1 ml of a 0.1 M carboxyfluorescein aqueous solution (adjusted to about pH 7) was added to 100 mg of a lipid mixture comprising dipalmitoyl phosphatidylcholine (DPPC)/cholesterol (CHOL)/maleimidocaproyldipalmitoyl phosphatidylethanolamine (JP-A-4-346918) in a molar ratio of 18/10/0.5 for hydration to obtain a multilamellar liposome. Further, particle size adjustment was carried out by a 0.1 µm polycarbonate membrane by using an extruder (LIpex Biomembranes).

Bonding of the antibody and the liposome was carried out by a reaction at 25°C for one hour in a 0.1 M citrate buffer solution of pH 6 at a final concentration of the antibody being 0.48 mg/ml and at the final concentration of the liposome (as lipid) being 19 mg/ml, and after the reaction, the product was subjected to gel permeation chromatography by Sepharose CL6B (Pharmacia) to separate and remove an unreacted antibody and a non-encapsulated carboxyfluorescein.

The activity of each immunoliposome thus prepared, was determined by measuring the amount bonded on a 96 well plastic plate having an antigen fixed, by measuring the quantity of fluorescence of the encapsulated carboxyfluorescein. Namely, 20 ug/ml of the antigen CEA was added to a 96 well plate (Falcon) in an amount of 50 µl/well and fixed at 37°C for two hours. After removing CEA, the plate was washed with PBS, followed by blocking with 5% bovine serum albumin. A liposome solution diluted to each concentration with 1% bovine serum albumin, after washing with PBS, was added to the plate in an amount of 50 µl/well and reacted at 4°C for 90 minutes. After thoroughly washing with PBS, a 2% triton × 100-containing PBS was added in an amount of 100 µl/well, followed by incubation at 37°C for 30 minutes, to elute carboxyfluorescein from the liposome. A part thereof was sampled and diluted with PBS, whereupon the fluorescence was measured at an emission wavelength of 520 nm with an excitation wavelength of 492 nm, and the amounts of liposomes bonded to the antigen plate were compared.

As a result, as shown in Figure 2, a high bonding activity was observed with the immunoliposome prepared by introducing SH to the antibody employing Ac-S-PEG-Suc.

### EXAMPLE 3

A complex was prepared by using fluorescein maleimido (Funakoshi) being a maleimido-containing fluorescent dye, instead of the liposome in Example 2. Namely, 5 µl of a DMSO solution containing 3.8 mg/ml of fluorescein maleimido was added to 0.35 ml of the 2.5 mg/ml SH-PEG-anti-CEA antibody (a 0.1 M phosphate buffer solution of pH 6.0, a 1 mM EDTA solution) as shown in Example 2, and reacted at 25°C for 30 minutes with stirring.

After the reaction, the product was subjected to gel permeation with PD-10 (Pharmacia) equillibrated with PBS to remove unreacted fluorescein maleimido. The introduced ratio of fluorescein maleimido was calculated from the absorbance at 495 nm and 280 nm, whereby 0.9 molecule of fluorescein maleimido per antibody was found to have been introduced. This fluorescent-labeled antibody was added to human gastric cancer cell line MKN45 and mixed in human blood serum at 50 µg/ml for one hour under cooling with ice. After thoroughly washing with PBS, bonding to the cancer cells was observed by a flow cytometer. As a result, as shown in Figure 3, it was confirmed that the complex was bonded to the cancer cells and exhibited fluorescence.

### EXAMPLE 4

In 1 ml of chloroform, 20 mg (4 µmol) of PEG disuccinimidyl succinate (Sunbright 4001, manufactured by Nippon Oil and Fat Co., Ltd., PEG average molecular weight: 5000) was dissolved. While stirring the PEG solution, 4 µmol of mercaptoethylamine hydrochloride (Sigma) dissolved in dehydrated methanol, was dropwise added thereto. Further, 56 µl of 72 mM triethylamine (in dehydrated methanol) was added. After a reaction in a nitrogen atmosphere at room temperature for 7 hours, consumption of an amino group was inspected by a ninhydrin reaction, whereby the ninhydrin reaction was negative and thus it was confirmed that it reacted with the disuccinimide PEG. The obtained SH-PEG-succinimide was evaporated to dryness in a nitrogen stream and redissolved in 1 ml of dehydrated methanol. Insoluble matters were filtered off, and the filtrate was used for the following reaction with an antibody.

48.7 µl of the PEG derivative was added to 0.5 ml of 3.9 mg/ml human γ-globulin dissolved in a 50 mM phosphate buffer solution of pH 7.5 and 1 mM EDTA with stirring and shaked at 37°C for one hour. Unreacted PEG derivative and antibody were separated by gel permeation chromatography employing Sephadex G75 column (1.2 cm × 12 cm) equillibrated with a 0.1 M phosphate buffer solution of pH 6.0 and 1 mM EDTA. By development with the same buffer solution, the firstly eluted antibody fraction was collected, and the amount of introduced thiol was quantitatively analyzed in the same manner as in Example 1. As a result, 0.75 mol of thiol was found introduced per mol of the antibody.

Further, bonding to a liposome was carried out in the same manner as in Example 2, and the obtained liposome was analyzed by SDS-PAGE, whereby bonding of the antibody was confirmed.

### EXAMPLE 5

### Comparison of leakage of carboxyfluorescein

Thiol-modified polyethylene glycol (JP-A-4-3461918) was further reacted to the antibody-bonded 0.1 M carboxyfluorescein-encapsulated liposome prepared in the same manner as in Example 2, to prepare an immunoliposome having the antibody and polyethylene glycol bonded thereto. The product was subjected to gel permeation by PD-10 column equillibrated with a 20 mM phosphate buffer solution and 0.3 M NaCl to remove non-encapsulated carboxyfluorescein. Further, the immunoliposome was incubated at 37°C, whereby the amount of carboxyfluorescein leaked from the liposome was measured.

The leakage of carboxyfluorescein was quantified by the method of Shahinian et al. (BBA 1239 (1995) 157). Namely, upon expiration of a predetermined time, a sample was taken from the liposome under incubation, and diluted with the same buffer solution, whereupon the quantity of fluorescence (excitation wavelength: 492 nm, emission wavelength: 520 nm) was measured. At the same time, the quantity of fluorescence after the liposome was added to 2% SDS solution and solubilized (destructed), was measured. The encapsulated carboxyfluorescein is in a self-quenching state, and the fluorescence intensity reflects the amount of carboxyfluorescein leaked from the liposome. Accordingly, the amount of leakage was calculated from the ratio of the two.

As shown in Figure 4, the antibody-PEG-liposome showed stability of at least equal to the non-modified liposome (EMC-liposome).

Shahinian et al. (BBA 1239 (1995) 157) have reported that a liposome having a maleimido group introduced at the distal end of PEG (maleimido-PEG-liposome or antibody bonded PEG-liposome) shows larger leakage than the liposome employing EMC-PE having no PEG moiety. Whereas, the liposome prepared by the method of the present invention showed stability of at least equal to the corresponding EMC-liposome.

### EXAMPLE 6

In the same manner as in Example 2, a carboxyfluorescein-encapsulated antibody-bonded liposome was prepared. Further, a thiol-modified polyethylene glycol prepared by using PEG having a molecular weight of 6,000 (JP-A-4-346918) was added to the liposome reaction solution. Namely, the thiol-modified polyethylene glycol dissolved in a 0.1 M phosphate buffer solution of pH 6.0 and 1 mM EDTA, was added in an amount of 2.5 µmol to 100 mg of the lipid and reacted at 10°C overnight. After the reaction, the product was subjected to gel permeation chromatography by Sepharose CL6B (Pharmacia) to separate and remove unreacted antibody, thiol-modified polyethylene glycol and non-encapsulated carboxyfluorescein.

Further, in the same manner as in Example 2, the amount bonded to a 96 well plastic plate having an antigen fixed, was determined by measuring the quantity of fluorescence of the encapsulated carboxyfluorescein. As a result, also with the antibody-bonded liposome coated with a thiol-modified polyethylene glycol, a high bonding activity was shown with the immunoliposome prepared by introducing SH to the antibody employing Ac-S-PEG-Suc, as shown in Figure 6.

### EXAMPLE 7

An adriamycin-encapsulated liposome having the antibody and PEG bonded (PEG immunoliposome) was prepared in the same manner as in Example 5 except that as the liposome, an adriamycin-encapsulated liposome as shown below, was used, and as the antibody, human IgG was used. As controls, a liposome having only thiol-modified PEG bonded (PEG liposome) and a liposome having none of them bonded (liposome) were prepared.

The adriamycin-encapsulated liposome was prepared in such a manner that 1 ml of a 0.3 M citrate buffer of pH 4.0 was added per 100 mg of a solid lipid mixture comprising dipalmitoyl phosphatidylcholine (DPPC)/cholesterol (CHOL) /maleimidocaproyldipalmitoyl phosphatidylethanolamine in a molar ratio of 18/10/0.5 and hydrated by a vortex mixer to prepare a multilameller liposome. Then, this liposome was extruded through polycarbonate membranes having pores sizes of 0.2 µm and 0.1 µm (LIpex Biomembranes) in turn under heating to 60°C to obtain a liposome having a particle size adjusted. The obtained liposome solution was neutralized with 1 M NaOH and then, adriamycin (Kyowa Hakko) in an amount of 1/10 by weight of the lipid weight was added to encapsulate at least 97% of adriamycin.

To compare retention in blood of the respective liposomes, 2 mg/kg as the amount of adriamycin was administered from the tale vein to a male BALB/c mouse. After each period of time, the mouse was killed, and the plasma was collected. The amount of adriamycin was quantitatively analyzed by measuring fluorescence after extraction with hydrochloric acid/ethanol in accordance with a method of Konno et al. (Cancer Res. 47 4471 (1987)).

Further, free adriamycin disappeared from blood swiftly after the administration, and is not detected under this condition. Accordingly, the amount of detected adriamycin is considered to reflect the behavior as the relevant liposome.

The results are shown in Figure 7, wherein the antibody prepared by this method and the PEG-bonded adriamycin-included liposome showed long circulating property in blood.

### EXAMPLE 8

The antitumor activity against tumor cells, of the immunoliposome employing the bifunctional water-soluble polymer derivative having adriamycin encapsulated, was examined in vitro. Using an example of a human antibody having reactivity to human colon cancer, the selective antitumor effect of the present immunoliposome against target cells was confirmed.

### 1-3-1 antibody reactive to colon cancer

Human monoclonal antibody 1-3-1 reactive to human colon cancer disclosed in JP-A-5-304987 developed in CHO cells, which was class-switched to IgG by a conventional method, was subjected to pepsin digestion (JP-A-4-346918) to use it as a F(ab')₂ fragment.

For the purpose of confirming the reactivity of an antibody, the above antibody fluorescence-labeled with FITC, was prepared, and the reactivities against human colon cancer cells DLD-1 (manufactured by Dainippon Seiyaku) and against human umbilical vascular endothelial cell-derived SV-3T cells (Agric. Biol. Chem. 55 (11), 2847-2853, 1991) were measured by a flow cytometer.

Namely, the fluorescent antibody was adjusted by a BSA solution (PBS containing 1% BSA) to be 50 µg/ml, then added to the cells and reacted for one hour under cooling with ice. After washing once with the BSA solution, propidium iodide (PI) in a final concentration of 2 µg/ml was added, followed by measurement by the flow cytometer (FACScan, Becton Dickinson).

Living cells i.e. PI negative cells were selected by a gating operation, and with respect to the average channel value representing the fluorescence intensity of FITC, a value obtained by subtracting, as a background value, the value where no antibody was contained in the respective cells, was shown in Figure 8.

It was confirmed that the 1-3-1 antibody showed higher reactivity against DLD-1 cells than against SV-3T cells.

### Preparation of immunoliposome

In the same manner as in Example 7, an adriamycin-encapsulated PEG immunoliposome was prepared.

### Cell proliferation inhibitory activity (Cytotoxicity)

The present immunoliposome was sequentially diluted with healthy human-derived blood serum to prepare a series of diluted system starting with 40 µg/ml as the concentration of adriamycin. Each sample was added to cells, followed by incubation at 37°C for one hour, then the cells were washed once with the culture medium and cultured at 37°C. When a control containing no liposome sample, became confluent, a MTT assay (J. Immunol. Methods, 65 55 (1983)) was carried out, and the numbers of cells were estimated.

The number of cells at each concentration of the liposome was shown as a relative value to the number of cells of the control being 100%.

Figure 9 shows the proliferation inhibitory activities against human DLD-1 cells and against SV-3T cells. The present immunoliposome showed a more specific inhibitory effect against the target colon cancer cells DLD-1. Namely, it is evident that the present immunoliposome has an antitumor effect.

If the bifunctional water-soluble polymer derivative of the present invention is used, a ligand-polymer-active substance complex can readily be obtained by mixing and reacting it with an active ligand and substance such as a liposome. With the water-soluble polymer derivative of the present invention, it is possible to prevent deterioration in the activity of the ligand or to prevent an adverse effect to an active substance.

According to the method of the present invention, a ligand such as an antibody is firstly bonded to a polymer derivative such as PEG and then bonded to a micelle, whereby it is possible to produce a ligand-bonded complex without introducing an excessive amount of a polymer derivative such as PEG to a thermodynamically unstable micelle and accordingly, without an adverse effect thereof.

Further, in the present invention, when a drug or the like is included in the micelle, it is possible to reduce leakage of the included substance, and a high bonding activity to a target molecule such as an antigen can be obtained.

## Claims

1. A bifunctional water-soluble polymer derivative as a spacer for binding to a ligand and an active substance selected from a liposome, the polymer derivative having a succinimide group as a moiety reactive with an amino group. and a S-acetylthio group moiety as a latent thiol group moiety.

2. The water-soluble polymer derivative according to Claim 1, which is represented by the following formula (I): 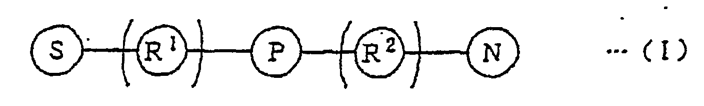 wherein S is said S-acetylthio group moiety, N is said succinimide group, P is a water-soluble polymer and each of R¹ and R² is an optional linking group, but R¹ and/or R² is not essential.

3. The polymer derivative according to Claim 1 or 2, wherein the water-soluble polymer is a biodegradable polymer or a polyethylene glycol.

4. The polymer derivative according to any one of Claim 1 to 3, wherein the water-soluble polymer is a polyethylene glycol.

5. A complex having a ligand and an active substance selected from a liposome, bonded via the polymer derivative as defined in any one of Claims 1 to 4.

6. The complex according to Claim 5, which is represented by the following formula (II): 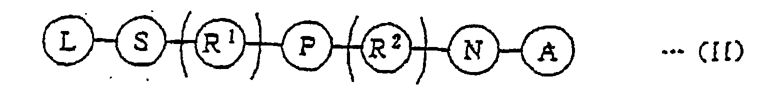 wherein L is said liposome, A is a ligand, and S, R¹, P, R² and N are as defined above.

7. The complex according to Claim 5 or 6, wherein the ligand is an antibody.

8. The complex according to Claim 5 or 6, wherein the ligand is an antibody 1-3-1.

9. The complex according to any one of the Claims 5 to 8, wherein a monofunctional water-soluble polymer derivative having reactivity with the liposome, is bonded to the liposome.

10. The complex according to any one of Claims 5 to 9, wherein a drug or a diagnostic agent is included in the liposome.

11. A pharmaceutical composition containing the complex as defined in any one of Claims 5 to 10.

12. The pharmaceutical composition according to Claim 11, which is used as an antitumor agent.

13. A diagnostic composition containing the complex as defined in any one of Claims 5 to 10.

14. A method for producing the complex as defined in any one of Claims 5 to 10, which comprises steps of bonding the bifunctional water-soluble polymer derivative as defined in any one of Claims 1 to 4, to a ligand and then bonding it, via the other end of the water-soluble polymer derivative, to a liposome.

15. The method according to Claim 14, which further contains a step of bonding a monofunctional water-soluble polymer derivative having reactivity with the liposome, to the liposome.

16. The method according to Claim 14 or 15, wherein the bifunctional water-soluble polymer derivative and the ligand are bonded via an amino group of the ligand

17. The method according to any one of the Claims 14 to 16, wherein the bifunctional water-soluble polymer derivative and the liposome are bonded such that a moiety reactive with a thiol group of the liposome is bonded to S-acetylthio group moiety of the bifunctional water-soluble polymer derivative.

## Patentansprüche

1. Bifunktionelles, wasserlösliches Polymerderivat als Spacer zur Bindung an einen Liganden und eine Wirkstoffsubstanz, gewählt aus einem Liposom, wobei das Polymerderivat eine Succinimidgruppe als eine mit einer Aminogruppe reaktiven Einheit und eine S-Acetylthiogruppeneinheit als eine latente Thiolgruppeneinheit besitzt.

2. Wasserlösliches Polymerderivat nach Anspruch 1, welches durch die folgende Formel (I) wiedergegeben wird: 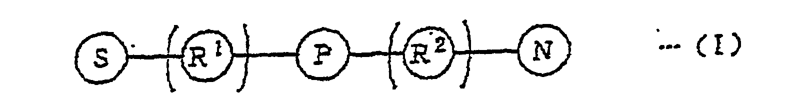 worin S die S-Acetylthiogruppeneinheit ist, N die Succinimidgruppe ist, P ein wasserlösliches Polymer ist und jedes von R¹ und R² ein wahlweise Verbindungsgruppe ist, wobei jedoch R¹ und/oder R² nicht wesentlich ist.

3. Polymerderivat nach Anspruch 1 oder 2, wobei das wasserlösliche Polymer ein bioabbaubares Polymer oder ein Polyethylenglykol ist.

4. Polymerderivat nach mindestens einem der Ansprüche 1 bis 3, wobei das wasserlösliche Polymer ein Polyethylenglykol ist.

5. Komplex mit einem Liganden und einer Wirkstoffsubstanz, gewählt aus einem Liposom, gebunden über ein Polymerderivat, wie in mindestens einem der Ansprüche 1 bis 4 definiert.

6. Komplex nach Anspruch 5, welcher durch die folgende Formel (II) wiedergegeben wird: 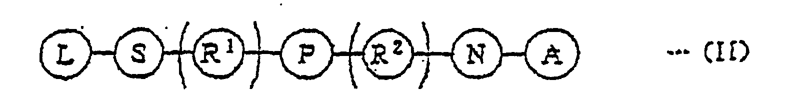 worin L das Liposom ist. A ein Ligand ist, und S, R¹, P, R² und N wie oben definiert sind.

7. Komplex nach Anspruch 5 oder 6, wobei der Ligand ein Antikörper ist.

8. Komplex nach Anspruch 5 oder 6, wobei der Ligand ein Antikörper 1-3-1 ist.

9. Komplex nach mindestens einem der Ansprüche 5 bis 8, wobei ein monofunktionelles, wasserlösliches Polymerderivat, das gegenüber dem Liposom reaktiv ist, an das Liposom gebunden ist.

10. Komplex nach mindestens einem der Ansprüche 5 bis 9, wobei ein Arzneimittel oder ein diagnostisches Mittel in dem Liposom beinhaltet ist.

11. Pharmazeutische Zusammensetzung, enthaltend den Komplex, wie in mindestens einem der Ansprüche 5 bis 10 definiert.

12. Pharmazeutische Zusammensetzung nach Anspruch 11, die als Antitumormittel verwendet wird.

13. Diagnostische Zusammensetzung, enthaltend den Komplex, wie in mindestens einem der Ansprüche 5 bis 10 definiert.

14. Verfahren zur Herstellung des in mindestens einem der Ansprüche 5 bis 10 definierten Komplexes, umfassend Schritte des Bindens des bifunktionellen, wasserlöslichen Polymerderivats, wie in mindestens einem der Ansprüche 1 bis 4 definiert, an einen Liganden und danach Binden dieses, über das andere Ende des wasserlöslichen Polymerderivats, an ein Liposom.

15. Verfahren nach Anspruch 14, enthaltend weiterhin einen Schritt des Bindens eines monofunktionellen, wasserlöslichen Polymerderivats, das gegenüber dem Liposom reaktiv ist, an das Liposom.

16. Verfahren nach Anspruch 14 oder 15, wobei das bifunktionelle, wasserlösliche Polymerderivat und der Ligand über eine Aminogruppe des Liganden gebunden werden.

17. Verfahren nach mindestens einem der Ansprüche 14 bis 16, wobei das bifunktionelle, wasserlösliche Polymerderivat und das Liposom so gebunden werden, dass eine mit einer Thiolgruppe des Liposoms reaktive Einheit an eine S-Acetylthiogruppeneinheit des bifunktionellen, wasserlöslichen Polymerderivats gebunden wird.

## Revendications

1. Dérivé polymère soluble dans l'eau bifonctionnel servant d'élément d'espacement pour une liaison à un ligand et une substance active choisie parmi les liposomes, le dérivé polymère ayant un groupe succinimide à titre de fragment réactif avec un groupe amino, et un fragment groupe S-acétylthio à titre de fragment groupe thiol latent.

2. Dérivé polymère soluble dans l'eau selon la revendication 1, qui est représenté par la formule (I) suivante : 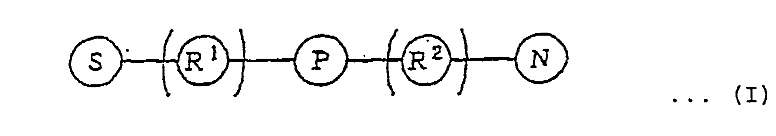 dans laquelle S est ledit fragment groupe S-acétylthio, N est ledit groupe succinimide, P est un polymère soluble dans l'eau et chacun de R¹ et R² est un groupe de liaison facultatif, mais R¹ et/ou R² ne sont pas essentiels.

3. Dérivé polymère selon la revendication 1 ou 2, dans lequel le polymère soluble dans l'eau est un polymère biodégradable ou un polyéthylèneglycol.

4. Dérivé polymère selon l'une quelconque des revendications 1 à 3, dans lequel le polymère soluble dans l'eau est un polyéthylèneglycol.

5. Complexe ayant un ligand et une substance active qui est un liposome, liés via le dérivé polymère tel que défini dans l'une quelconque des revendications 1 à 4.

6. Complexe selon la revendication 5, qui est représenté par la formule (II) suivante : 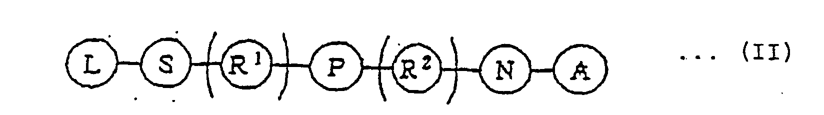 dans laquelle L est ledit liposome, A est un ligand, et S, R¹, P, R² et N sont tels que définis ci-dessus.

7. Complexe selon la revendication 5 ou 6, dans lequel le ligand est un anticorps.

8. Complexe selon la revendication 5 ou 6, dans lequel le ligand est un anticorps 1-3-1.

9. Complexe selon l'une quelconque des revendications 5 à 8, dans lequel un dérivé polymère soluble dans l'eau monofonctionnel ayant une réactivité vis-à-vis du liposome est lié au liposome.

10. Complexe selon l'une quelconque des revendications 5 à 9, dans lequel un médicament ou un agent de diagnostic est incorporé dans le liposome.

11. Composition pharmaceutique contenant le complexe tel que défini dans l'une quelconque des revendications 5 à 10.

12. Composition pharmaceutique selon la revendication 11, qui est utilisée en tant qu'agent antitumoral.

13. Composition de diagnostic contenant le complexe tel que défini dans l'une quelconque des revendications 5 à 10.

14. Procédé pour produire le complexe tel que défini dans l'une quelconque des revendications 5 à 10, qui comprend les étapes consistant à lier le dérivé polymère soluble dans l'eau bifonctionnel tel que défini dans l'une quelconque des revendications 1 à 4 à un ligand et ensuite à le lier, par l'autre extrémité du dérivé polymère soluble dans l'eau, à un liposome.

15. Procédé selon la revendication 14, qui comprend en outre une étape consistant à lier au liposome un dérivé polymère soluble dans l'eau monofonctionnel ayant une réactivité vis-à-vis du liposome.

16. Procédé selon la revendication 14 ou 15, dans lequel le dérivé polymère soluble dans l'eau bifonctionnel et le ligand sont liés via un groupe amino du ligand.

17. Procédé selon l'une quelconque des revendications 14 à 16, dans lequel le dérivé polymère soluble dans l'eau bifonctionnel et le liposome sont liés de façon qu'un fragment réactif avec un groupe thiol du liposome soit lié à un fragment groupe S-acétylthio du dérivé polymère soluble dans l'eau bifonctionnel.
